# EUROPEAN PATENT APPLICATION

(11) **EP 4 447 065 A1**
(43) Date of publication of application: **16.10.2024**
(21) Application number: 24169446.2
(22) Date of filing: 10.04.2024
(51) Int. Cl.: G16H 20/17, A61M 1/00, G16H 20/40, G16H 40/63

(54) **DEVICE SCREEN INTERACTION USING A SCANNER AND BIDIRECTIONAL DATA TRANSFER**

(30) Priority: 12.04.2023 US 202363458759 P
(71) Applicant: Fenwal, Inc., Lake Zurich, IL 60047 (US)
(72) Inventor: KUSTERS, Benjamin E., Lake Zurich, 60047 (US); MIN, Kyungyoon, Lake Zurich, 60047 (US); MADSEN, James G., Lake Zurich, 60047 (US)
(74) Representative: Maikowski & Ninnemann Patentanwälte Partnerschaft mbB

(57) **Abstract**

A biological fluid processing system and a method of executing a biological fluid processing procedure is disclosed. The biological fluid processing system includes a processing device, a scanner, and a data management system. The processing device includes a display screen and a controller which operates the processing device to implement a biological fluid processing procedure having a plurality of steps. The controller displays an image on the display screen. The scanner interacts with the device screen to retrieve data from the image and transmit the data to the data management system. The scanner and/or the data management system transmit a signal to the controller based on the data. The controller initiates at least one step of the biological fluid processing procedure upon receiving the signal.

## Description

### Background

### Field of the Disclosure

The present disclosure generally relates to processing of biological fluids using a processing device having a display screen. More particularly, the present disclosure relates to scanning an image on a processing device screen to initiate a step of a biological fluid processing procedure.

### Description of Related Art

Using various manual and automated systems and methods, whole blood may be collected and separated into its clinical components (typically red blood cells, platelets, and plasma). The collected components are typically individually stored and used to treat a variety of specific conditions and diseased states. These components can be stored or transported in a container or bag.

Means of identification can be affixed to the containers or bags holding blood or blood components. This identification can be helpful when an end user (e.g., a hospital) receives the storage containers. Different types of identification labels, such as barcodes or radio-frequency identification ("RFID") tags may be used as identifiers. These identifiers are not only applied to biological fluid containers, but they are also commonly applied to containers for non-biological fluids (e.g., an anticoagulant fluid, an additive solution, saline, etc.) and other disposable components used in combination with a durable processing device during a procedure (e.g., a fluid flow circuit or kit). Identifiers may also be used to identify a participant in a biological fluid processing procedure (e.g., an operator or technician may be provided with an identification card bearing a barcode or RFID tag that is scanned by a scanner or reader of the processing device).

The manner in which data is retrieved from an identifier will vary according to the nature of the identifier. For example, barcode scanners are used to retrieve data from a barcode, while RFID readers are used to retrieve data from an RFID tag. When data is retrieved from an identifier in connection with execution of a biological fluid processing procedure, it is transmitted to an associated data management system, with the data being used by the data management system for various purposes, such as confirming that a selected fluid flow circuit is appropriate for use with a procedure being implemented by the processing device. Notably, this transfer of data is unidirectional - the data contained in the identifier is transmitted to the data management system, where it is stored or analyzed or manipulated or otherwise used by the data management system.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a biological fluid processing system includes a processing device, a scanner, and a data management system. The processing device includes a display screen and a controller configured to operate the processing device to implement a biological fluid processing procedure having a plurality of steps. The controller is configured to control the display screen to display an image. The scanner is configured to interact with the device screen to retrieve data from the image and to transmit the data to the data management system. The scanner and/or the data management system is configured to transmit a signal to the controller based on the data. The controller is configured to control the processing device to initiate at least one step of the biological fluid processing procedure upon receiving the signal.

In another aspect, a method of executing a biological fluid processing procedure using a biological fluid processing system including a processing device, a scanner, and a data management system includes displaying an image on a display screen of the processing device, scanning the image with the scanner to retrieve data from the image, and transmitting the data from the scanner to the data management system. The method further includes transmitting a signal based on the data from the scanner and/or from the data management system to the processing device so as to cause the processing device to initiate at least one step of the biological fluid processing procedure upon receiving the signal.

### Brief Description of the Drawings

Fig. 1 is a perspective view of an exemplary reusable hardware component or processing device of a biological fluid processing system which is configured to receive a disposable fluid flow circuit;
Fig. 2 is a schematic view of a disposable fluid flow circuit suitable for use with the processing device shown in Fig. 1;
Fig. 3 is a schematic view of an exemplary biological fluid processing system according to an aspect of the present disclosure; and
Figs. 4-8 illustrate exemplary images that may be presented on a display screen of a biological fluid processing device during a biological fluid processing procedure.

### Description of the Illustrated Embodiments

The embodiments disclosed herein are for the purpose of providing a description of the present subject matter, and it is understood that the subject matter may be embodied in various other forms and combinations not shown in detail. Therefore, specific designs and features disclosed herein are not to be interpreted as limiting the subject matter as defined in the accompanying claims.

The current disclosure includes biological fluid processing systems and methods which employ an image displayed on a screen of a biological fluid processing device for the bidirectional transfer of data between the processing device and a scanner and/or a data management system. As will be described in greater detail herein, retrieving data from the image using a scanner leads to the transmission of a signal from the scanner and/or the data management system to a controller of the biological fluid processing device, with the controller initiating at least one step of a biological fluid processing procedure upon receiving the signal. Such bidirectional transfer of data alleviates the need for manually initiating a step of the biological fluid processing procedure (e.g., requiring an operator or technician to press a button or interact with the display screen or another data entry device to instruct the controller to initiate a step of the procedure).

Biological fluid processing systems according to the present disclosure include a durable or reusable biological fluid processing device, a scanner or reader, and a data management system. A disposable or single-use fluid flow circuit is mounted to the processing device during a biological fluid processing procedure, with the fluid flow circuit being dissociated from the processing device at the end of a procedure.

The reusable processing device may be any apparatus that can provide for the automated processing of biological fluid. As used herein, the term "biological fluid" encompasses (without limitation) whole blood drawn from a living source that is connected to the processing device during a procedure, previously collected whole blood drawn from a container during a procedure, and previously separated blood components (e.g., packed red blood cells or a platelet product) drawn from a container during a procedure. A "biological fluid" may include a non-biological fluid (e.g., whole blood including an anticoagulant fluid or a separated blood component including a storage or additive solution) without departing from the scope of the present disclosure.

As used herein, the term "automated" means that the processing device can be programmed to carry out the various steps of a biological fluid processing procedure without substantial operator involvement. Of course, even in automated systems of the type described herein, it will be understood that operator activity may be involved, including the loading and unloading of a disposable fluid circuit and the scanning of an image on a display screen.

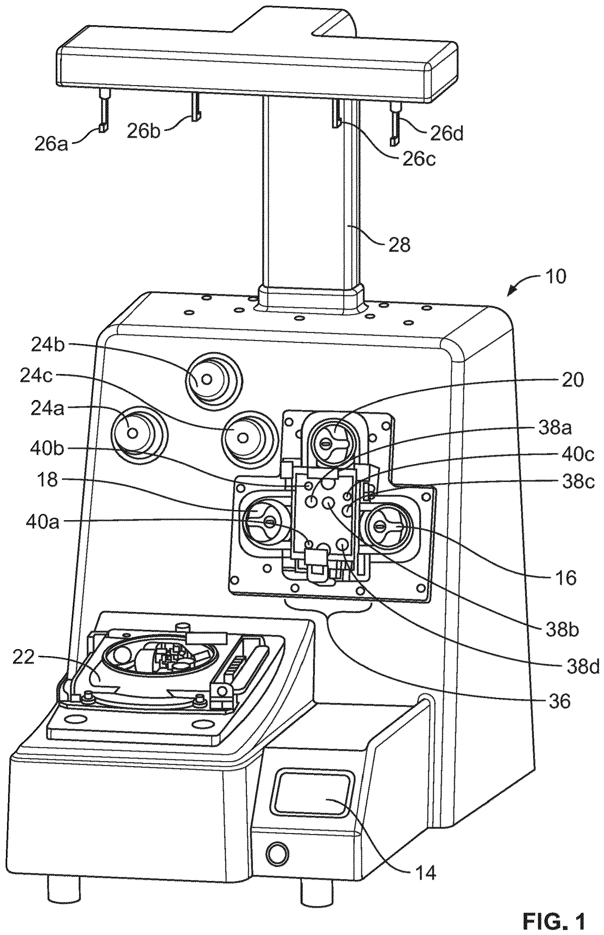

Fig. 1 depicts one example of a reusable hardware component or processing device 10 of a biological fluid processing system according to an aspect of the present disclosure. The processing device 10 described herein is configured to execute a variety of biological fluid processing procedures, including the separation of a biological fluid (e.g., whole blood) into two or more constituents (e.g., packed red blood cells and a plasma constituent). However, it should be understood that the present disclosure is not limited to any particularly configured processing device or any particular biological fluid processing procedure, but encompasses any suitably configured processing device and any procedure in which a biological fluid is treated or processed in some way. Exemplary biological fluid processing procedures include separation of a biological fluid into two or more components (which will be described herein in greater detail), washing of a blood component (e.g., red blood cells), or irradiation of a blood component (e.g., a platelet product). While the processing device described herein is capable of executing a variety of different biological fluid processing procedures, it is within the scope of the present disclosure for a processing device to be configured to execute only one type of biological fluid processing procedure.

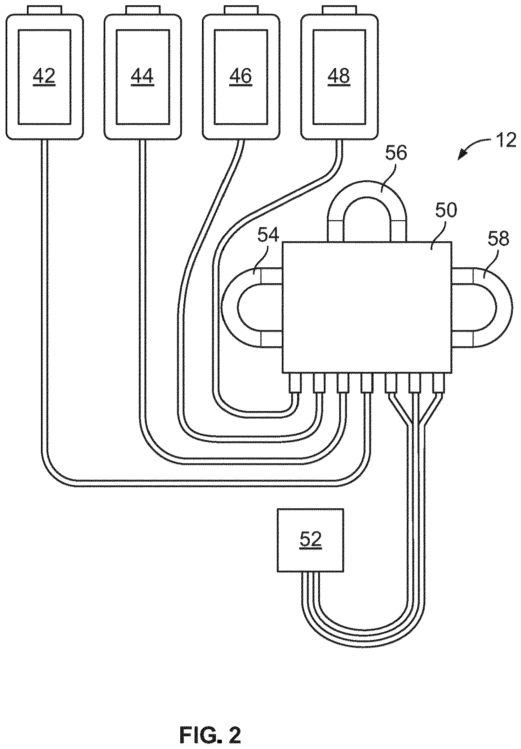

Fig. 2 depicts a disposable fluid flow circuit, generally designated 12, that is suitable to be used in combination with the illustrated processing device 10 for processing a biological fluid. The basic configuration and operation of the illustrated processing device 10 and fluid flow circuit 12 may be better understood with reference to PCT Patent Application Publication No. WO 2021/194824 A1, which is hereby incorporated herein by reference. It should be understood that the illustrated fluid flow circuit 12 of Fig. 2 is merely exemplary and that the present disclosure encompasses differently configured fluid flow circuits, with the configuration of a fluid flow circuit being dependent upon the configuration of the processing device to which the fluid flow circuit is to be mounted. For example, as will be described in greater detail herein, the illustrated processing device 10 includes three pumps, with the fluid flow circuit 12 including components (namely, flexible tubing loops) that are positioned and configured to be manipulated by the pumps of the processing device 10. A fluid flow circuit used in combination with a processing device having a different number of pumps and/or differently configured pumps and/or differently positioned pumps will be differently configured than the fluid flow circuit 12 of Fig. 12, but suitably configured to complement the configuration of the associated processing device.

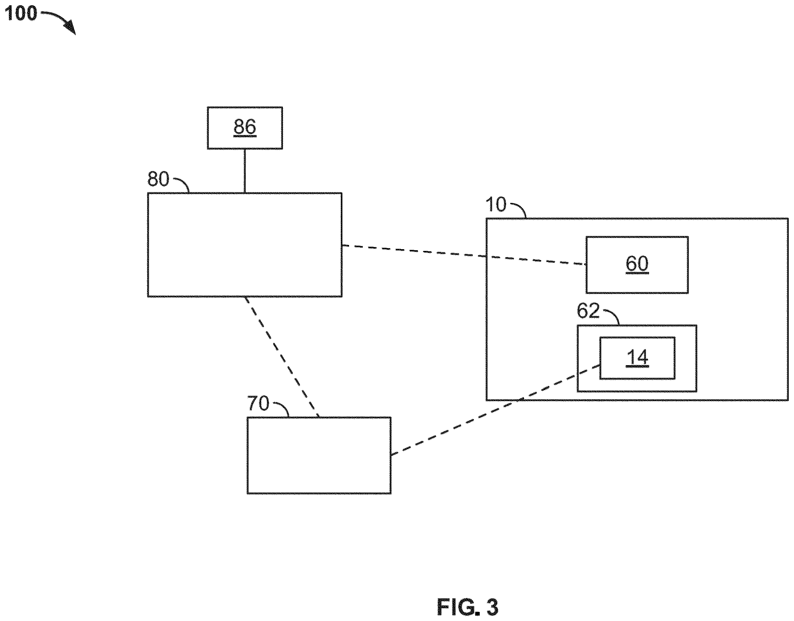

Turning now more particularly to the configuration of the illustrated processing device 10, it includes a display screen 14; a pump station including a first pump 16 (for pumping, e.g., whole blood), a second pump 18 (for pumping, e.g., plasma), and a third pump 20 (for pumping, e.g., an additive solution); a centrifuge mounting station and drive unit 22 (which may be referred to herein as a "centrifuge"); and clamps 24a-c. The display screen 14 (which will be described in greater detail herein) may enable user interaction with a controller 60 of the processing device 10 (Fig. 3), which may be pre-programmed to automatically operate the processing device 10 to perform one or more biological fluid processing procedures selected by an operator, with the controller 60 also carrying out various other functions during a procedure (e.g., monitoring of procedure parameters, such as flow rates, container weights, pressures, etc.). The pumps 16, 18, and 20 (collectively referred to herein as being part of a "pump system" of the processing device 10) are illustrated as peristaltic pumps capable of receiving tubing or conduits of the fluid flow circuit 12 and moving fluid at various rates through the associated conduit. An exemplary centrifuge mounting station/drive unit is described in U.S. Patent No. 8,075,468 (with reference to Figs. 26-28), which is hereby incorporated herein by reference. The clamps 24a-c (collectively referred to herein as being part of the "valve system" of the processing device 10) are capable of opening and closing fluid paths through the tubing or conduits of the fluid flow circuit 12 and may incorporate RF sealers in order to complete a heat seal of the tubing or conduit placed in the clamp to seal the tubing or conduit leading to a product container upon completion of a procedure.

The illustrated processing device 10 also includes hangers 26a-d (which may each be associated with a weight scale) for suspending the various containers of the disposable fluid circuit 12. The hangers 26a-d may be mounted to a support 28, which is vertically translatable to improve the transportability of the processing device 10.

The face of the processing device 10 includes a nesting module 36 for seating a flow control cassette 50 (Fig. 2) of the fluid flow circuit 12. The cassette nesting module 36 is configured to receive various disposable cassette designs so that the system may be used to perform different types of biological fluid processing procedures. Embedded within the illustrated cassette nesting module 36 are four valves 38a-d (collectively referred to herein as being part of the "valve system" of the processing device 10) for opening and closing fluid flow paths within the flow control cassette 50, and three pressure sensors 40a-c capable of measuring the pressure at various locations of the flow control cassette 50.

The illustrated flow control cassette 50 is fluidically connected to the other components of the fluid flow circuit 12 by conduits that may be configured as flexible tubing. The conduits or tubing may extend through the cassette 50, or the cassette 50 may have pre-formed internal fluid flow paths that direct the fluid flow. With reference to Fig. 2, the other components of illustrated fluid flow circuit 12 include a plurality of containers 42, 44, 46, and 48 and a processing/separation chamber 52 that is configured to be received in the centrifuge 22. In addition to these illustrated components, the fluid flow circuit 12 may include a variety of other features and/or components (e.g., an air trap and/or a leukoreduction filter) without departing from the scope of the present disclosure.

The cassette 50 provides a centralized, programmable, integrated platform for all the pumping and valving functions required for a given biological fluid processing procedure. In use, the cassette 50 is mounted to the cassette nesting module 36 of the processing device 10. The valves 38a-38d of the module 36 apply positive and negative pneumatic pressure to corresponding valve chambers of the cassette 50 to control and direct fluid flow therethrough, while the pumps 16, 18, and 20 act upon tubing loops 54, 56, and 58 extending from the cassette 50 to convey fluid into and out of the cassette 50. The operation of the pumps and valves to control fluid flow through the cassette 50 can be understood with reference to U.S. Patent Application Publication No. 2009/0215602, which is hereby incorporated herein by reference.

The containers 42, 44, 46, and 48 of the fluid flow circuit 12 may be configured to hold a variety of different biological and non-biological fluids, depending on the nature of the procedure to be executed. In one exemplary embodiment, container 42 of the fluid flow circuit 12 shown in Fig. 2 may be pre-filled with an additive solution, container 44 may be filled with whole blood and connected to the fluid flow circuit 12 at the time of use, container 46 may be an empty container for the receipt of red blood cells separated from the whole blood, and container 48 may be an empty container for the receipt of plasma separated from the whole blood. While Fig. 2 shows a container 44 (configured as a blood pack unit, for example) as a biological fluid source, it is within the scope of the present disclosure for the biological fluid source to be a living source (e.g., a donor or patient). Further, while the illustrated fluid flow circuit 12 includes three containers, it should be understood that a different number of containers may be provided without departing from the scope of the present disclosure.

It is again emphasized that the illustrated processing device 10 and associated fluid flow circuit 12 are merely exemplary and may be differently configured (e.g., using pneumatic pumps instead of peristaltic pumps and/or a spinning membrane separator instead of a centrifugal separation chamber) without departing from the scope of the present disclosure.

As noted above, the processing device 10 includes a controller 60, which is shown in FIG 3. The controller 60 may be variously configured without departing from the scope of the present disclosure, provided that it is configured to coordinate the various tasks carried out by the components of the processing device 10 during a biological fluid processing procedure. In one embodiment, the controller 60 may include a microprocessor (which, in fact may include multiple physical and/or virtual processors). According to other embodiments, the controller 60 may include one or more electrical circuits designed to carry out the actions described herein. In fact, the controller 60 may include a microprocessor and other circuits or circuitry. In addition, the controller 60 may include one or more memories. The instructions by which the microprocessor is programmed may be stored on the memory associated with the microprocessor, which memory/memories may include one or more tangible non-transitory computer readable memories, having computer executable instructions stored thereon, which when executed by the microprocessor, may cause the microprocessor to carry out one or more actions as described herein. In one exemplary embodiment, the controller 60 comprises a main processing unit (MPU), which can comprise, e.g., a PENTIUM^{®} type microprocessor made by Intel Corporation, although other types of conventional microprocessors can be used.

The controller 60 may be coupled to one or more of the components of the processing device 10 to receive information (e.g., in the form of signals) from these components and/or to provide commands (e.g., in the form of signals) to these components to control the operation of the components. For example, the controller 60 may be coupled to the scales, sensors, and centrifuge to provide commands to those components to control their operation. The controller 60 may be directly electrically connected to these other components to be coupled to them, or the controller 60 may be directly connected to other intermediate equipment that is directly connected to these components to be coupled to them. The controller 60 is also configured to communicate with one or more other devices of a biological fluid processing system 100 such as devices associated with a data management system 80 and a scanner or reader 70 (Fig. 3).

Among the various components of the processing device 10 coupled to the controller 60 is an input or data entry device 62. The data entry device 62 may be differently configured without departing from the scope of the present disclosure. For example, the data entry device 62 may include a keyboard or keypad by which a user may provide information and/or instructions to the controller 60. The data entry device 62 may also include a reader or scanner, such as a barcode reader or scanner or an RFID reader. According to still other embodiments, the data entry device 62 may be in the form of computer equipment that permits the processing device 10 to communicate (whether via wires, cables, etc. or wirelessly) with other processing systems or devices over a local network, or with other processing systems or other computer equipment (e.g., a server) over local networks, wide area networks, or the Internet or with the data management system 80. According to such an embodiment, the data entry device 62 may include an internal transmitter/receiver device.

In the embodiment shown in Fig. 3, the display screen 14 is a component of the data entry device 62. In this configuration, the screen 14 (which may be configured as an interactive touch screen and which may be positioned as shown in Fig. 1 or at some other location of the processing device 10) allows an operator to interact with the controller 60 of the device 10 to provide instructions to the controller 60 (e.g., selecting a step of a procedure to be executed), as well as providing information to the controller 60 to be used during a procedure (e.g., information regarding the biological fluid source). Alternatively, rather than allowing for an operator or technician to interact with the controller 60, the display screen 14 may be more simply configured, providing instructions to an operator for carrying out a procedure (e.g., to connect or disconnect a source from the fluid flow circuit 12) and/or information about a procedure (e.g., alerting the operator to a blockage in a conduit of the fluid flow circuit 12). Indeed, it should be understood that the display screen 14 may be variously configured (e.g., being a component of the data entry device 62 or being unaffiliated with the data entry device 62) without departing from the scope of the present disclosure, provided that it is configured as a video display capable of displaying or presenting an image that can be interacted with by a scanner or reader 70.

Turning now to the other components of the biological fluid processing system 100 (Fig. 3), a data management system 80 is provided for communication with the controller 60 of the processing device 10. The data management system 80 may be variously configured without departing from the scope of the present disclosure, provided that it is capable of communicating with the controller 60 of the processing device 10 and with the scanner or reader 70 (as will be described in greater detail herein). In various embodiments, the data management system 80 may include a server, be accessible via "the cloud" or a web-based network, or otherwise be configured to receive and transmit data and signals via any other suitable communication protocol.

In one embodiment, the data management system 80 is able to store information from the processing device 10 and any other connected processing devices (which may be differently configured). The data management system 80 may provide the ability to view, analyze, and/or track data related to biological fluid processing procedures. For instance, the data management system 80 may be configured to receive from the processing device 10 procedure data relating to a procedure performed by the processing device 10. The procedure data may comprise operator interactions with the processing device 10 that the processing device 10 has sensed or otherwise determined have occurred. The procedure data may additionally (or alternatively) comprise information about disposable components (e.g., soft goods or other consumable materials) used in the procedure, such as a lot number, reference number, expiration date, product code, type, size, or other data about a disposable component. The procedure data may additionally (or alternatively) comprise an indication of an operation performed by the processing device 10. The procedure data may additionally (or alternatively) comprise a system notification, alarm, or alert generated by a processing device 10 to provide information to an operator of the occurrence of an event (e.g., improper loading of a disposable component, operation completion, operator interaction required, etc.). The procedure data may additionally (or alternatively) comprise an identifier of one or more persons who have controlled the processing device 10 to perform an operation and may further comprise an indication of their authorization level.

In addition, or in the alternative, the data management system 80 may provide the ability to view, analyze, manipulate, and/or track data separate and apart from such procedure data. This may include the data management system 80 being configured to remotely alter (or to allow a user to alter) the operation of an associated processing device 10. For example, and as will be elaborated further below, a user at the processing device 10 or at an associated computing device 86 may start or stop a procedure (or a step thereof) or modify a parameter of a procedure.

The data stored by the data management system 80 can be accessed by an operator when operating the processing device 10 or by any user via an appropriately configured data access device 86. This may include a system 100 having a plurality of data access devices 86 capable of interacting with the data management system 80. A data access device 86 may be variously configured without departing from the scope of the present disclosure, provided that it is capable of communicating with the data management system 80 (via a wired or wireless network connection or email or the like). For example, a data access device 86 may be configured as a personal computer, tablet, laptop, or cellular phone. By nature, the processing device 10, data management system 80, and data access device 86 do not need to be physically connected, but rather may be disposed in separate rooms, buildings or laboratories, or even in different cities, countries, etc.

The data provided to the data management system 80 may be provided in real time and accessible by a user at a data access device 86 or a processing device 10 within a short time of when an event has occurred. The data management system 80 may also be configured to present at least some procedure data and/or instrument status (e.g., idle, in process, stopped, etc.) from a processing device 10 on a display of a data access device 86 to allow a user to remotely monitor the processing device 10.

Access to the data management system 80 may be restricted such that only certain users have the ability to access the system, and may require authorization. This may occur at the associated data access device 86, prompted by the data management system 80. The data management system 80 may be configured to determine if a user has authorization that permits the user to enter and/or modify the process parameters (and/or to enter and/or modify process parameters within a controlled range) and/or instruct a processing device 10 to start or stop a step of a procedure. This determination may be made any time a user attempts to access information or to input information using the data management system 80. Authorization may be provided based on an identifier provided by a user, such as an alphanumeric password or passcode or a two-dimensional or three-dimensional barcode or RFID tag applied to a badge or key that is read by a data access device 86. Other possible embodiments also exist.

In addition to communicating with the controller 60 of a processing device 10, the data management system 80 also communicates with a scanner or reader 70 of the system 100 (Fig. 3). The scanner 70 may also communicate with the controller 60, with communication of these various components of the system being bidirectional, as will be described in greater detail herein. Communication between the scanner 70 and the other components of the system 100 may be instantaneous, via a wired connection (e.g., if the scanner 70 is connected to the processing device 10 by a cord) or wireless connection (e.g., if the scanner 70 is separately provided from the processing device 10).

The scanner 70 is configured to retrieve data from an image or identifier displayed or presented on the screen 14 of a processing device 10 and to transmit the data to the data management system 80. The configuration of the scanner 70 will depend upon the nature of the image displayed by the screen 14 of the processing device 10. For example, if the screen 14 is configured to display a one- or two-dimensional barcode, the scanner 70 may have barcode scanning functionality, which may include the scanner 70 being configured as a portable or handheld barcode scanner or as another scanning device, such as a smartphone with an application or feature that provides the ability to scan a barcode. While use of an image or identifier configured as a barcode may be advantageous, it should be understood that differently configured images or identifiers may be displayed by the screen 14 of a processing device 14, provided that relevant data may be communicated to the data management system 80 upon the image or identifier being read by the scanner 70.

Now that the various components of an exemplary biological fluid processing system 100 according to the present disclosure have been described, an exemplary biological fluid processing procedure using the system 100 will now be described. It is once again emphasized that the present disclosure is not limited to any particular biological fluid processing procedure, with the procedure hereafter described being merely exemplary.

Regardless of the exact nature of a biological fluid processing procedure, it typically includes an operator or technician loading disposable components (e.g., a fluid flow circuit 12) onto the processing device 10. The controller 60 of the processing device 10 may be provided with preliminary information and data by the technician (e.g., manually entering procedure parameters (e.g., a target volume of biological fluid to be processed) or information regarding a fluid source (e.g., platelet pre-count) using the data entry device 62) and/or by the scanner 70 (operated by a technician) and/or by the data management system 80 (either acting automatically or by operation of a user). This preliminary information and data may include selection of a procedure from among the variety of procedures that the device 10 is capable of performing.

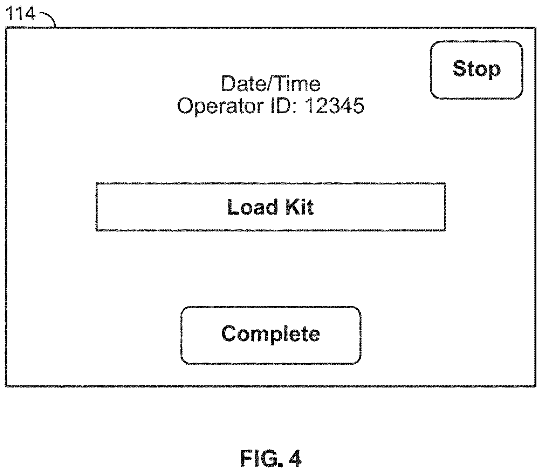

When the controller 60 has received all of the necessary input, it may instruct the video screen 14 to display a message on the video screen 14 to instruct the operator to mount a disposable kit or fluid flow circuit 12 to the processing device 10 (if not already mounted to the device 10). An exemplary "load kit" graphic 114 that may be displayed on the screen 14 is shown in Fig. 4. The operator loads the kit 12 (if not already loaded) and then informs the controller 60 (e.g., by pressing a "complete" icon on the screen 14) that the kit 12 has been loaded to proceed to the next screen. Upon the operator indicating that the kit 12 has been loaded onto the device 10, the controller 60 may execute a pre-procedure routine to confirm that the various components of the device 10 are functioning properly and that an appropriate kit 12 is being used.

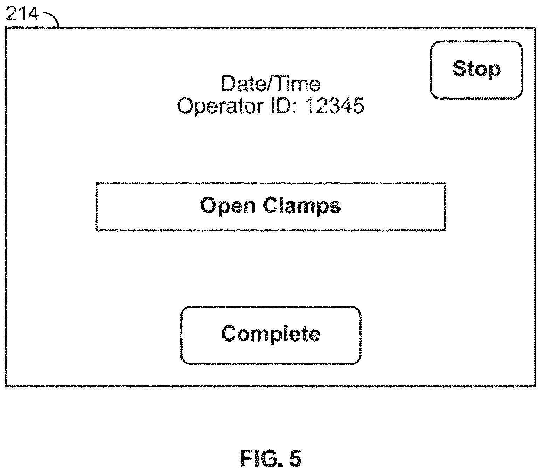

After the kit 12 has been loaded, the controller 60 may instruct the video screen 14 to display a subsequent message instructing the operator to carry out any other preliminary steps that must be manually performed, such as opening manual clamps on the tubing lines of the kit 12. An exemplary "open clamps" graphic 214 that may be displayed on the screen 14 is shown in Fig. 5. The operator opens the appropriate clamps and then informs the controller 60 (e.g., by pressing a "complete" icon on the screen 14) that the clamps have been opened to proceed to the next screen. Upon the operator indicating that the clamps have been opened, the controller 60 may execute a routine to confirm that the proper clamps have been opened and that it is appropriate to proceed to the next stage of the procedure.

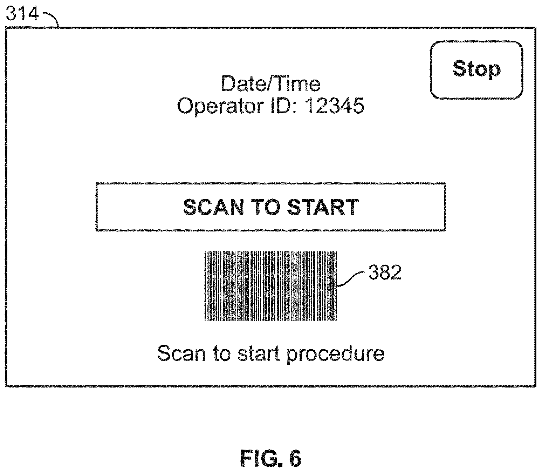

After the initial manual operations have been completed, the processing device 10 is ready to begin processing a biological fluid. At this time, the controller 60 controls the video screen 14 to display an icon to be scanned by the operator using the scanner 70. An exemplary "scan to start" graphic 314 and image or identifier 382 are shown in Fig. 6. While the image 382 is shown in Fig. 6 as a one-dimensional barcode, it is again emphasized that the image 382 may be variously configured without departing from the scope of the present disclosure (provided that it is capable of providing the necessary information to the scanner 70). Notably, the image 382 is only displayed on the video screen 14 at the appropriate time (i.e., when the automated portion of a biological fluid processing procedure is to begin), rather than the image being permanently affixed to a portion of the device 10 or a component of the fluid flow circuit 12 or being continuously presented on the screen 14. This approach prevents the image 382 from being scanned at an inappropriate time.

Scanning of the image 382 on the screen 14 using the scanner 70 will cause the scanner 70 to transfer various data to the data management system 80. This data may include (without limitation) information regarding the configuration of the fluid flow circuit 12, the identity of the operator, and the nature of the process to be executed by the processing device 10.

Scanning the image 382 also causes the data management system 80 and/or the scanner 70 to send a signal to the controller 60 of the processing device 10. The signal (which is based on at least a portion of the data communicated to the scanner 70 by the image 382) instructs the controller 60 of the device 10 to start the automated portion of the selected biological fluid processing procedure. The procedure will then automatically begin without any further input from the operator (e.g., pressing a "start procedure" icon or button, as is required by conventional biological fluid processing devices).

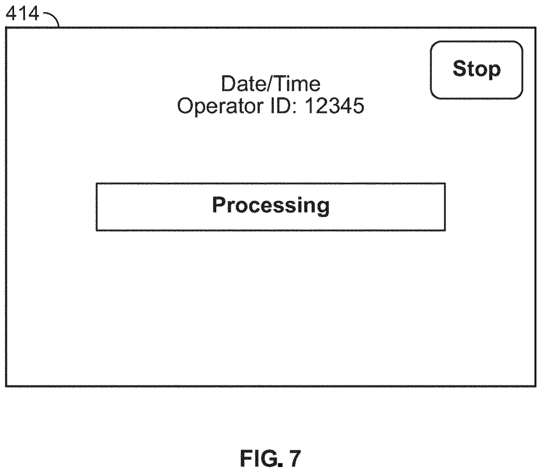

Once the image 382 on the screen 14 has been scanned and the processing device 10 has begun the automated portion of the procedure, the controller 60 may control the screen 14 to display a "processing" graphic 414, such as the one shown in Fig. 7. This graphic 414 may change during the course of a procedure, informing the operator of the step currently being performed by the device 10 (e.g., blood draw, separation, fluid return, etc.).

Once the procedure is complete, the controller 60 may control the screen 14 to inform the operator and instruct the operator to remove the fluid flow circuit 12 (including any collected fluid components) from the processing device 10.

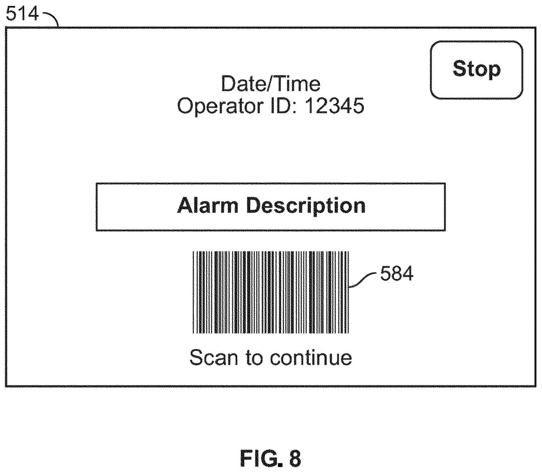

If, at any time during a procedure, there is an error that causes the procedure to be stopped or temporarily paused, the controller 60 may control the video screen 14 to display an error or alarm message. An exemplary "alarm" graphic 514 is shown in Fig. 8. The "alarm" graphic 514 may include another image or identifier 584 that is scanned by an operator using the scanner 70 (after the controller 60 and/or the operator have taken the necessary steps to address any error conditions) to continue the procedure (similar to the initial image 382 that is scanned using the scanner 70 to begin the automated portion of the procedure). So creating a bidirectional event that both restarts the device 10 and transfers data to the data management system 80 allows for a record to be made of various aspects of the error condition.

While the images or identifiers 382 and 584 are described as being scanned using the scanner 70 to begin the automated portion of a procedure (in the case of image 382) or to restart a paused procedure (in the case of image 584), it should be understood that a similar image or identifier may be displayed or presented on the video screen 14 in place of any icon displayed on the screen 14 or other element of the data entry device 62 (e.g., a button) that is conventionally used to initiate one or more individual steps of a biological fluid processing procedure. For example, an initial image 382 may be scanned using the scanner 70 to begin the first step of the automated portion of a procedure (e.g., a kit priming stage), with a subsequent image being displayed and then scanned using the scanner 70 to initiate a second step of the procedure (e.g., a blood draw stage), a third image being displayed and then scanned using the scanner 70 to initiate a later step of the procedure (e.g., a leukoreduction stage), and so on. This may include a final image or identifier that is scanned using the scanner 70 at the end of a procedure to transfer any relevant data to the data management system 80 and to instruct the controller 60 to power off the processing device 10 (or to perform any other step that is appropriate upon completion of a procedure).

Thus, an improved method and system have been disclosed for the processing of blood components. The description provided above is intended for illustrative purposes only and is not intended to limit the scope of the invention to any specific method, system, or apparatus, or device described herein except as may be explicitly delineated above.

### Aspects

Aspect 1. A biological fluid processing system, comprising: a processing device including a display screen and a controller configured to operate the processing device to implement a biological fluid processing procedure having a plurality of steps; a scanner; and a data management system, wherein the controller is configured to control the display screen to display an image, the scanner is configured to interact with the device screen to retrieve data from the image and to transmit the data to the data management system, the scanner and/or the data management system is configured to transmit a signal to the controller based on the data, and the controller is configured to control the processing device to initiate at least one step of the biological fluid processing procedure upon receiving the signal.

Aspect 2. The biological fluid processing system of Aspect 1, wherein the image is a barcode and the scanner is a barcode scanner.

Aspect 3. The biological fluid processing system of Aspect 2, wherein the barcode is a one-dimensional barcode.

Aspect 4. The biological fluid processing system of Aspect 2, wherein the barcode is a two-dimensional barcode.

Aspect 5. The biological fluid processing system of any one of the preceding Aspects, wherein the image is configured to include data regarding a fluid flow circuit used in combination with the processing device during the biological fluid processing procedure.

Aspect 6. The biological fluid processing system of any one of the preceding Aspects, wherein the image is configured to include data regarding an operator or technician operating the processing device.

Aspect 7. The biological fluid processing system of any one of the preceding Aspects, wherein the controller is configured to initiate an automated portion of the biological fluid processing procedure upon receiving the signal.

Aspect 8. The biological fluid processing system of any one of Aspects 1-6, wherein the controller is configured to restart a paused biological fluid processing procedure upon receiving the signal.

Aspect 9. The biological fluid processing system of any one of the preceding Aspects, wherein the display screen is configured as an interactive touch screen.

Aspect 10. The biological fluid processing system of any one of Aspects 1-8, wherein the display screen is not configured as an interactive touch screen.

Aspect 11. A method of executing a biological fluid processing procedure using a biological fluid processing system including a processing device, a scanner, and a data management system, the method comprising: displaying an image on a display screen of the processing device; scanning the image with the scanner to retrieve data from the image; transmitting the data from the scanner to the data management system; and transmitting a signal based on the data from the scanner and/or from the data management system to the processing device so as to cause the processing device to initiate at least one step of the biological fluid processing procedure upon receiving the signal.

Aspect 12. The method of Aspect 11, wherein the image is a barcode and the scanner is a barcode scanner.

Aspect 13. The method of Aspect 12, wherein the barcode is a one-dimensional barcode.

Aspect 14. The method of Aspect 12, wherein the barcode is a two-dimensional barcode.

Aspect 15. The method of any one of Aspects 11-14, wherein the image is configured to include data regarding a fluid flow circuit used in combination with the processing device during the biological fluid processing procedure.

Aspect 16. The method of any one of Aspects 11-15, wherein the image is configured to include data regarding an operator or technician operating the processing device.

Aspect 17. The method of any one of Aspects 11-16, wherein the processing device is configured to initiate an automated portion of the biological fluid processing procedure upon receiving the signal.

Aspect 18. The method of any one of Aspects 11-16, wherein the processing device is configured to restart a paused biological fluid processing procedure upon receiving the signal.

Aspect 19. The method of any one of Aspects 11-18, wherein the display screen is configured as an interactive touch screen.

Aspect 20. The method of any one of Aspects 11-18, wherein the display screen is not configured as an interactive touch screen.

It will be understood that the embodiments and examples described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A biological fluid processing system, comprising:
a processing device including a display screen and a controller configured to operate the processing device to implement a biological fluid processing procedure having a plurality of steps;
a scanner; and
a data management system, wherein
the controller is configured to control the display screen to display an image,
the scanner is configured to interact with the device screen to retrieve data from the image and to transmit the data to the data management system,
the scanner and/or the data management system is configured to transmit a signal to the controller based on the data, and
the controller is configured to control the processing device to initiate at least one step of the biological fluid processing procedure upon receiving the signal.

2. The biological fluid processing system of claim 1, wherein the image is a barcode and the scanner is a barcode scanner, preferably the barcode is a one-dimensional barcode or a two-dimensional barcode.

3. The biological fluid processing system of any one of the preceding claims, wherein the image is configured to include data regarding a fluid flow circuit used in combination with the processing device during the biological fluid processing procedure.

4. The biological fluid processing system of any one of the preceding claims, wherein the image is configured to include data regarding an operator or technician operating the processing device.

5. The biological fluid processing system of any one of the preceding claims, wherein the controller is configured to initiate an automated portion of the biological fluid processing procedure upon receiving the signal.

6. The biological fluid processing system of any one of claims 1-4, wherein the controller is configured to restart a paused biological fluid processing procedure upon receiving the signal.

7. The biological fluid processing system of any one of the preceding claims, wherein the display screen is configured as an interactive touch screen.

8. The biological fluid processing system of any one of claims 1-6, wherein the display screen is not configured as an interactive touch screen.

9. A method of executing a biological fluid processing procedure using a biological fluid processing system including a processing device, a scanner, and a data management system, the method comprising:
displaying an image on a display screen of the processing device;
scanning the image with the scanner to retrieve data from the image;
transmitting the data from the scanner to the data management system; and
transmitting a signal based on the data from the scanner and/or from the data management system to the processing device so as to cause the processing device to initiate at least one step of the biological fluid processing procedure upon receiving the signal.

10. The method of claim 9, wherein the image is a barcode and the scanner is a barcode scanner, preferably the barcode is a one-dimensional barcode or
a two-dimensional barcode.

11. The method of any one of claims 9-10, wherein the image is configured to include data regarding a fluid flow circuit used in combination with the processing device during the biological fluid processing procedure.

12. The method of any one of claims 9-11, wherein the image is configured to include data regarding an operator or technician operating the processing device.

13. The method of any one of claims 9-12, wherein the processing device is configured to initiate an automated portion of the biological fluid processing procedure upon receiving the signal.

14. The method of any one of claims 9-12, wherein the processing device is configured to restart a paused biological fluid processing procedure upon receiving the signal.

15. The method of any one of claims 9-14, wherein the display screen is configured as an interactive touch screen or wherein the display screen is not configured as an interactive touch screen.
